# FASCICULE DE BREVET EUROPEEN

(11) **EP 1 626 666 B1**
(45) Date de publication et mention de la délivrance du brevet: **20.12.2006**
(21) Numéro de dépôt: 04767206.8
(22) Date de dépôt: 28.05.2004
(51) Int. Cl.: A61B 17/70

(54) **DISPOSITIF DE CONNEXION POUR OSTEOSYNTHESE RACHIDIENNE**
VERBINDUNGSVORRICHTUNG FÜR WIRBELSÄULENOSTEOSYNTHESE
CONNECTING DEVICE FOR SPINAL OSTEOSYNTHESIS

(30) Priorité: 28.05.2003 FR 0306523
(43) Date de publication de la demande: 22.02.2006
(73) Titulaire: Spinevision, 75012 Paris (FR)
(72) Inventeur: BETTE, Stéphane, F-75011 Paris (FR); PETIT, Dominique, F-62180 Verton (FR); THIBOUT, Didier, F-75020 Paris (FR); VANACKER, Gérard, F-94100 Saint-Maur (FR)
(74) Mandataire: Breese Derambure Majerowicz
(86) Numéro de dépôt international: PCT/FR2004/001330
(87) Numéro de publication internationale: WO 2004/107997

(56) Documents cités:
- DE-A- 4 107 480
- US-A- 5 443 467
- US-A- 5 752 957

## Description

La présente invention se rapporte au domaine de l'ostéosynthèse rachidienne destinée à la chirurgie du rachis pour la correction et la stabilisation de la colonne vertébrale.

La présente invention se rapporte plus particulièrement à un dispositif de connexion pour l'ostéosynthèse rachidienne comprenant un moyen d'ancrage osseux, un connecteur destiné à recevoir une tige de liaison et assemblé avec une extrémité du moyen d'ancrage, ainsi qu'un moyen de serrage, pour immobiliser la tige de liaison.

Il est connu dans l'art antérieur de nombreux systèmes pour corriger ou stabiliser la colonne vertébrale

Il est proposé dans le certificat d'utilité allemand DE4107480 une vis à os destinée à la fixation d'un dispositif pour corriger et stabiliser la colonne vertébrale. Pour ce faire, ladite vis à os est constituée d'une tête présentant une zone évidée semicirculaire destinée à recevoir la tige de liaison. Ladite tige de liaison est bloquée sur la tête de la vis à os par une pièce de fermeture en forme sensiblement de U, ladite pièce de fermeture formant pince sur la tête de ladite vis.

Le problème qui se pose avec cette solution est que la tige n'est pas stabilisée dans le basculement dans le plan contenant la tige de liaison et passant par la vis à os.

Il est également proposé, dans la demande de brevet européen EP0614649 une vis à os comprenant un élément vis présentant une partie filetée et une tête qui possède une partie en forme de segment sphérique, et une pièce réceptrice cylindrique destinée à recevoir la tête de l'élément vis et une barre qui doit être assemblée à la vis à os. Ladite pièce réceptrice présente, à une de ses extrémités, un premier perçage destiné à laisser passer la partie filetée de l'élément vis, une partie de forme sphérique creuse destinée à l'application de la tête, un second perçage ouvert formé à l'opposé du premier perçage et destiné à l'introduction de la partie fileté munie de la tête. Ladite pièce réceptrice présente en outre une section sensiblement en forme de U possédant deux branches libres munies d'un filetage extérieur et un filetage intérieur, et dans laquelle sont prévus un élément de pression agissant sur la tête, une vis de coincement vissée sur le côté ouvert, au-dessus de la barre destinée à être placée dans la section en forme de U, et un écrou de blocage vissé sur le filetage extérieur des branches de ladite pièce réceptrice.

Les dispositifs de l'art antérieur présentent cependant des inconvénients. D'une part, les mouvements permis sur les vis à os restent relativement faibles en particulier dans le plan frontal. Il en découle alors des difficultés pour manipuler, et notamment monter ou démonter les tiges de liaison sur les vis à os. D'autre part, lors de la chirurgie de reprise, le dévissage de la vis à os peut s'avérer difficile du fait d'un non-alignement du moyen d'entraînement de la vis avec la tête réceptrice de la tige de liaison.

La présente invention entend remédier aux inconvénients de l'art antérieur en proposant un dispositif de connexion permettant d'augmenter le débattement du connecteur maintenant la tige de liaison, sur le moyen d'ancrage utilisé sur les vertèbres.

La présente invention a pour but ainsi d'offrir un dispositif permettant une manipulation aisée, et plus particulière de faciliter le montage et démontage de la tige de liaison sur le moyen d'ancrage, ainsi que de faciliter le dévissage du moyen d'ancrage lors de l'opération de chirurgie de reprise.

Pour ce faire, la présente invention est du type décrit ci-dessus et elle est remarquable, dans son acception la plus large, en ce que l'assemblage du connecteur et du moyen d'ancrage est assuré par un axe traversant le moyen d'ancrage, ledit axe constituant une liaison pivot, et en ce que le dispositif comporte un moyen de stabilisation, dans le plan contenant la tige et le moyen d'ancrage, du connecteur par rapport au moyen d'ancrage lorsqu'il est implanté sur le patient.

De préférence, le connecteur coopère avec ledit axe avec un degré de rotation par rapport à la direction axiale, et un débattement angulaire par rapport à au moins une direction perpendiculaire.

Avantageusement, le connecteur coopère avec ledit axe avec une amplitude de rotation par rapport à la direction axiale comprise entre 90 et 180° et l'amplitude du débattement angulaire par rapport à au moins une direction perpendiculaire est comprise entre 20 et 60°.

De préférence, la partie de l'axe destinée à être disposée dans ledit moyen d'ancrage présente une convexité répartie uniformément sur la circonférence dudit axe (6) pour permettre un débattement angulaire dudit axe (6) sur ledit moyen d'ancrage par rapport à une direction perpendiculaire audit axe.

Selon un premier mode de réalisation de l'invention, ledit moyen de stabilisation est constitué par le fait que ledit axe présente une direction axiale parallèle à la direction axiale de ladite tige de liaison, autorisant un degré de rotation axial seulement, à l'exclusion d'un basculement dans le plan perpendiculaire à la tige.

Avantageusement, la partie de l'axe destinée à être disposée dans ledit moyen d'ancrage présente une convexité répartie uniformément sur la circonférence dudit axe pour permettre un débattement angulaire sagittal dudit axe sur ledit moyen d'ancrage.

Selon une première variante du premier mode de réalisation de l'invention, ledit connecteur comporte une surface d'appui sur laquelle la tige de liaison s'appuie, ladite surface d'appui étant configurée de manière à permettre un mouvement angulaire sagittal supplémentaire de ladite tige de liaison par rapport audit moyen d'ancrage.

Avantageusement, ladite surface d'appui est de forme bombée ou consiste en un élément formant berceau disposé dans le connecteur, ledit élément formant berceau présentant un logement inférieur coopérant avec le connecteur de manière à permettre le mouvement angulaire sagittal supplémentaire, et un logement supérieur destiné à recevoir la tige de liaison.

Selon une seconde variante du premier mode de réalisation de l'invention, le connecteur est traversé par un élément intermédiaire recevant ladite tige de liaison (2), ledit élément intermédiaire traversant présentant un logement inférieur coopérant avec l'extrémité dudit moyen d'ancrage de manière à permettre le verrouillage de tous les degrés de liberté de rotation dudit dispositif.

Avantageusement, ledit élément intermédiaire traversant présente une face supérieure de forme bombée permettant le mouvement angulaire sagittal de ladite tige de liaison par rapport audit connecteur ou consiste en un élément traversant formant berceau permettant le mouvement angulaire sagittal de ladite tige de liaison par rapport audit connecteur.

Avantageusement, le logement inférieur dudit élément intermédiaire traversant présente au moins une zone crantée ou striée. De même, l'extrémité du moyen d'ancrage est sur tout ou partie crantée ou striée.

Selon un second mode de réalisation de l'invention, ledit moyen de stabilisation est constitué par le fait que ledit axe présente une direction axiale perpendiculaire à la direction axiale de ladite tige de liaison et en ce que le dispositif comprend des moyens de blocage pour bloquer le mouvement de pivotement du connecteur sur ledit moyen d'ancrage.

De préférence, la partie de l'axe destinée à être disposée dans ledit moyen d'ancrage présente une convexité répartie uniformément sur la circonférence dudit axe pour permettre un débattement angulaire frontal dudit axe sur ledit moyen d'ancrage.

Avantageusement, les moyens de blocage consistent en une pièce traversant le connecteur, ladite pièce traversante présentant une face inférieure constituée d'un logement coopérant avec l'extrémité dudit moyen d'ancrage et une face supérieure recevant ladite tige de liaison, de manière à permettre le verrouillage de tous les degrés de liberté de rotation dudit dispositif. Dans un premier exemple de réalisation, la pièce traversante présente une face supérieure de forme bombée. Dans un autre exemple de réalisation, la pièce traversante consiste en un élément formant berceau lequel présente sur sa face supérieure un logement recevant ladite tige de liaison.

Avantageusement, le logement de la face inférieure de ladite pièce traversante est au moins en partie crantée ou striée. De même, l'extrémité dudit moyens d'ancrage en contact avec la pièce traversante est au moins en partie crantée ou striée.

Avantageusement, ledit axe présente sur tout ou partie de sa surface des stries longitudinales.

Avantageusement, ledit axe traverse l'extrémité du moyen d'ancrage et coopère avec deux trous prévus dans des branches du connecteur lesquels présentent des sections complémentaires à la section de l'axe (6), lesdites branches étant disposées de part et d'autre dudit axe.

Avantageusement, le moyen d'ancrage est une vis laquelle présente une partie filetée et une tête sphérique pourvue d'une cavité pour recevoir en partie ledit axe cylindrique.

Avantageusement, le connecteur est constitué d'une pièce réceptrice en forme sensiblement de U destinée à recevoir une extrémité dudit moyen d'ancrage pourvu de l'axe et la tige de liaison, et d'une pièce de fermeture en forme de U inversé, ladite pièce de fermeture venant s'emboîter sur la pièce réceptrice pour maintenir la tige de liaison sur la partie réceptrice dudit connecteur par l'intermédiaire dudit moyen de serrage.

La présente invention se rapporte également à un système pour ostéosynthèse sur la colonne vertébrale, en particulier pour la stabilisation des vertèbres comprenant au moins une tige de liaison et au moins deux dispositifs de connexion selon l'une quelconque des revendications précédentes, lesdits dispositifs de connexion étant susceptibles d'être chacun ancrés dans une vertèbre.

On comprendra mieux l'invention à l'aide de la description, faite ci-après à titre purement explicatif, d'un mode de réalisation de l'invention, en référence aux figures annexées :
1. la figure 1 illustre une vue éclatée en perspective d'un dispositif de connexion et d'une tige de liaison selon un premier mode de réalisation de l'invention
2. la figure 2 illustre une vue en perspective du dispositif de connexion et de la tige de liaison de la figure 1 assemblés ;
3. la figure 3 illustre une vue en coupe en perspective du dispositif de connexion de la figure 2 avec la tige de liaison ;
4. la figure 4 illustre une vue de côté du dispositif de connexion sans la tige de liaison, ledit dispositif de connexion présentant un débattement angulaire frontal ;
5. la figure 5 illustre une vue en coupe axiale de face du dispositif de connexion et de la tige de liaison de la figure 2 ;
6. la figure 6 illustre une vue en coupe axiale de face du dispositif de connexion de la figure 5 pourvu de la tige de liaison, présentant un premier débattement angulaire sagittal ;
7. la figure 7 illustre une vue en coupe axiale de face du dispositif de connexion de la figure 5 pourvu de la tige de liaison, ledit dispositif de connexion présentant un débattement angulaire sagittal maximum ;
8. les figures 8 et 9 illustrent une vue en perspective d'un élément formant berceau du dispositif de connexion respectivement selon deux variantes de réalisation de l'invention ;
9. la figure 10 illustre une vue en perspective d'un axe traversant du dispositif de connexion selon une variante de l'invention ;
10. les figures 11 et 12 illustrent une vue en perspective d'un moyen d'ancrage du dispositif de connexion selon deux variantes de réalisation de l'invention ;
11. la figure 13 illustre une vue en coupe en perspective du dispositif de connexion pourvu d'une tige de liaison selon un autre mode de réalisation de l'invention.

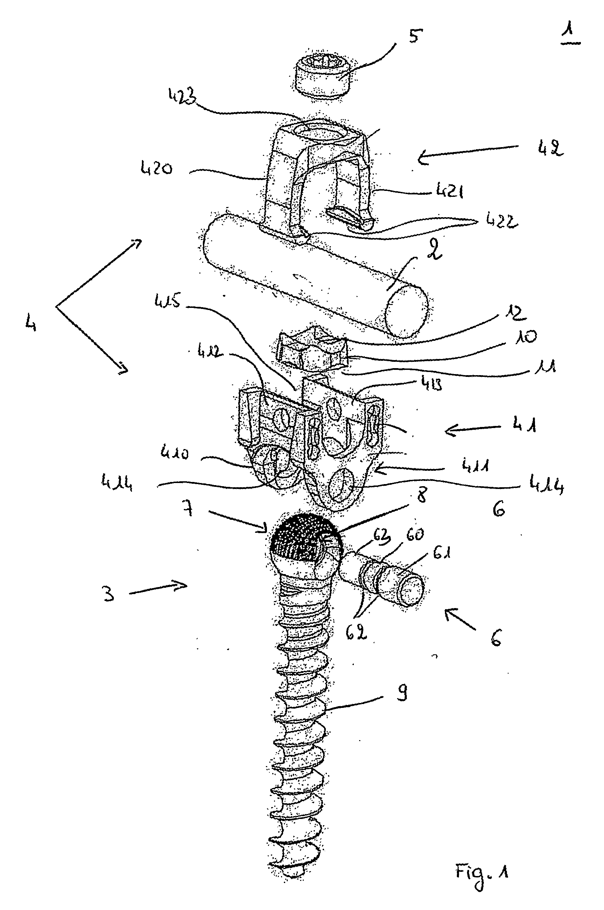

La figure 1 illustre une vue éclatée d'un dispositif (1) de connexion pour l'ostéosynthèse rachidienne selon l'invention. Ledit dispositif de connexion (1) est représenté avec une tige de liaison (2) de vertèbres.

Ledit dispositif de connexion (1) comprend un moyen d'ancrage osseux (3) et un connecteur (4) de tige de liaison (2). Ledit dispositif (1) comprend en outre un moyen de serrage (5) pour immobiliser le moyen d'ancrage (3) et le connecteur (4) maintenant la tige de liaison (2).

Avantageusement, ledit moyen d'ancrage osseux (3) consiste en une vis osseuse (3) constituée d'une partie filetée (9) et, à l'une des extrémités, d'une tête sphérique (7). La tête (7) de ladite vis (3) est pourvue d'une cavité (8) destinée à être traversée par un axe (6) cylindrique. La liaison entre la cavité (8) et l'axe (6) est réalisée de façon à permettre un mouvement de rotation dudit axe (6) dans ladite cavité (8). Pour ce faire, l'homme du métier pourra en particulier jouer sur les dimensions de la cavité (8) formée dans la tête (7) de la vis (3) et/ou dudit axe (6). Par exemple, des chanfreins d'entrée pourront être réalisés dans la cavité (8) de la tête (7) de la vis (3) afin de permettre un débattement angulaire dudit axe (6) accru.

En outre, il sera également possible d'utiliser un axe (6) présentant une surface non plane comme on le verra plus loin dans la description afin de permettre de contrôler le mouvement entre ledit axe (6) et ladite cavité (8).

Ledit axe (6) présente avantageusement sur sa circonférence une portion centrale (60) bombée. Ladite portion centrale (60) est séparée de part et d'autre des portions d'extrémité (61, 63) par une gorge (62). Ainsi, lorsque l'axe (6) est positionné au travers de la cavité (8) formée dans la tête (7) de la vis (3), la portion centrale (60) est disposée dans ladite cavité (8).

Le connecteur (4), destiné à connecter la tige de liaison (2) à ladite vis (3) comprend deux pièces, une pièce réceptrice (41) et une pièce de fermeture (42).

La pièce réceptrice (41) présente sensiblement une forme de U. Ladite pièce (41) est constituée en particulier de deux branches (410, 411) sensiblement en forme de chape. Lesdites branches (410, 411), disposées symétriquement l'une à l'autre, sont jointes l'une à l'autre par deux parois de raccordement (412, 413) latérales.

L'extrémité inférieure de chacune desdites branches (410, 411) est pourvue respectivement d'un trou (414), lesdits trous (414) présentant des sections complémentaires à la section de l'axe (6), pour être avantageusement monté serré sur le connecteur (4).

L'écartement entre chacune des extrémités desdites branches (410, 411) munies desdits trous (414) définit un espacement suffisant pour recevoir la tête (7) de ladite vis (3) lors de l'assemblage de la tige de liaison (2) sur la vis (3) par l'intermédiaire dudit connecteur (4) et ce, quelle que soit la position angulaire de ladite vis (3).

L'espacement supérieur défini par lesdites branches (410, 411) en forme de U et les parois de raccordement (412, 413) définit un logement (415) de réception pour la tige de liaison (2).

La pièce de fermeture (42) dudit connecteur (4) est de forme générale en U dont l'extrémité des branches (420, 421) est pourvue respectivement d'un épaulement (422) arqué vers l'intérieur desdites branches (420, 421) en U. Les dimensions et les formes des branches (420, 421) et des épaulements (422) sont déterminées de façon à permettre la mise en place et l'emboîtement de la pièce de fermeture (42) sur la pièce réceptrice (41).

La pièce de fermeture (42) présente sur le fond formé par les branches (420, 421), un logement (423). Avantageusement, ledit logement (423) comporte un taraudage (non représenté) pour recevoir ledit moyen de serrage (5), lequel permet le verrouillage définitif de ladite pièce de fermeture (42) par serrage dudit moyen de serrage (5).

Un élément intermédiaire traversant (10) formant berceauest disposé entre la pièce réceptrice (41) et la tige de liaison (2). Ledit élément berceau (10) présente sur sa face inférieure un logement (11) concave, ledit logement (11) étant dimensionné pour coopérer avec la tête de la vis (3). De la même manière, ledit élément intermédiaire traversant (10) présente sur sa face supérieure, laquelle est destinée à recevoir ladite tige de liaison (2), un second logement (12) concave, ledit logement (12) étant dimensionné pour coopérer avec ladite tige de liaison (2).

Le verrouillage du dispositif ainsi constitué est opéré par l'intermédiaire des forces exercées entre chacun des éléments. Ainsi, le moyen de serrage (5) exerce une pression sur la tige de liaison (2), qui elle-même exerce une pression sur l'élément intermédiaire traversant (10), lui-même exerçant une pression sur le moyen d'ancrage osseux (3). L'axe (6) traversant la tête (7) de ladite vis (3) exerce alors une traction sur la pièce réceptrice (41) qui elle-même exerce une traction sur la pièce de fermeture (42) par l'intermédiaire des épaulements (422) de la pièce de fermeture (42) se fixant dans des ogives formées dans la pièce réceptrice (41), la pièce de fermeture exerçant à son tour une traction sur le moyen de serrage (5) par l'intermédiaire du logement (423) fileté,

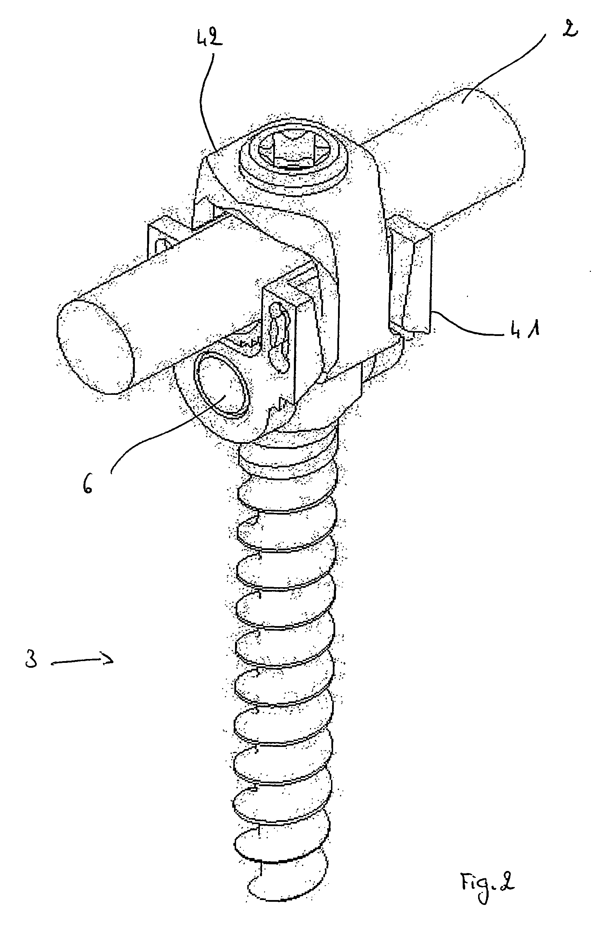

La figure 2 illustre une vue en perspective du dispositif de connexion (1) et de la tige de liaison (2) de la figure 1 assemblés.

Le connecteur (4) est assemblé avec la tête (7) de la vis (3) par l'intermédiaire de l'axe (6), lequel traverse la tête (7) de ladite vis (3) et coopère avec les deux trous (414) prévus dans les branches (410, 411) du connecteur (4), lesdites branches (410, 411) étant disposées de part et d'autre dudit axe (6).

La pièce de fermeture (42) dudit connecteur (4) est positionnée sur la pièce réceptrice (41) complémentaire de telle sorte que la face intérieure des branches (420, 421) de ladite pièce de fermeture (42) soit en contact avec les parois de raccordement (412, 413) de ladite partie réceptrice (41). La pièce de fermeture (42) refermée sur ladite pièce réceptrice (41) supportant la tige de liaison (2), ladite tige de liaison (2) est alors maintenue et associée avec la vis (3).

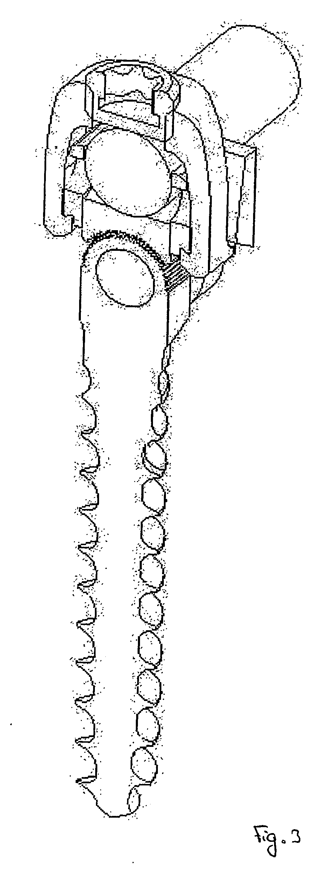

L'interaction entre chacun des éléments constituant le dispositif de connexion (1) selon l'invention est représenté sur la figure 3 laquelle illustre une vue en coupe dudit dispositif de connexion (1) assemblé sur la tige de liaison (2).

En particulier, il apparaît sur la figure 3 le blocage de la pièce de fermeture (42) sur la pièce réceptrice (41) dudit connecteur (4). Le blocage est réalisé par l'intermédiaire des épaulements (422) de la pièce de fermeture (42) lesquels sont insérés dans des ogives formées sur les parois de raccordement (412, 413) de ladite pièce réceptrice (41)

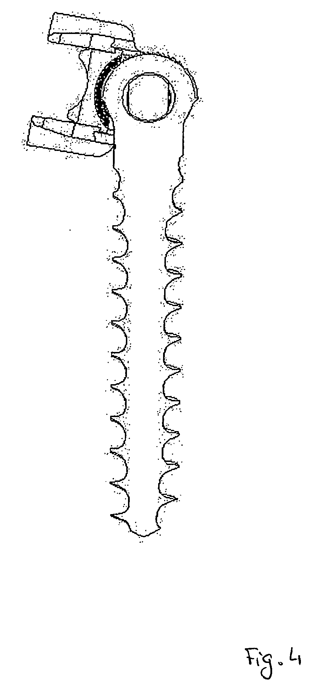

Le dispositif de connexion (1) ainsi assemblé permet d'obtenir du connecteur (4), lequel maintient fermement la tige de liaison (2), un mouvement de pivotement par rapport à ladite vis (3) selon deux directions, à savoir : un premier mouvement de pivotement parallèle à la direction axiale de la tige de liaison (2) (débattement frontal dudit connecteur (4) sur ladite vis (3)) et un mouvement de pivotement selon un axe perpendiculaire à la direction axiale de ladite tige de liaison (2) (débattement sagittal dudit connecteur (4) sur ladite vis (3)), ce dernier pouvant être augmenté d'un mouvement de pivotement latéral supplémentaire. Ces différents mouvements sont illustrés sur les figures 4 à 7.

En particulier, le débattement frontal dudit connecteur (4) sur ladite vis (3) est montré sur la figure 4, laquelle illustre une vue en coupe latérale dudit dispositif de connexion (1) où, pour une meilleure compréhension du fonctionnement, seuls la pièce réceptrice (41) dudit connecteur (4), l'élément intermédiaire traversant (10) et ladite vis (3) sont représentés.

Comme expliqué précédemment, de par les dimensions et la forme respectivement de la cavité (8) formée au niveau de la tête (7) de ladite vis (3) et dudit axe (6), il est possible de générer audit axe (6) un mouvement de rotation perpendiculaire à l'axe de la tige de liaison (2) (débattement sagittal). Et la partie réceptrice (41) dudit connecteur (4), fixée sur ledit axe (6), pivote sur la tête (7) de la vis (3). L'élément intermédiaire traversant (10), de par sa configuration, suit le pivotement de la partie réceptrice (41) par rapport à ladite vis (3) en glissant sur la tête (7) de ladite vis (3) sur laquelle ledit élément intermédiaire traversant (10) repose.

Avantageusement, le débattement frontal peut atteindre 180 degrés.

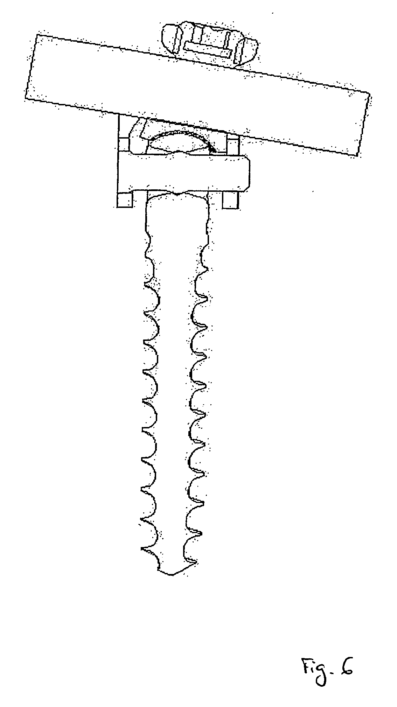

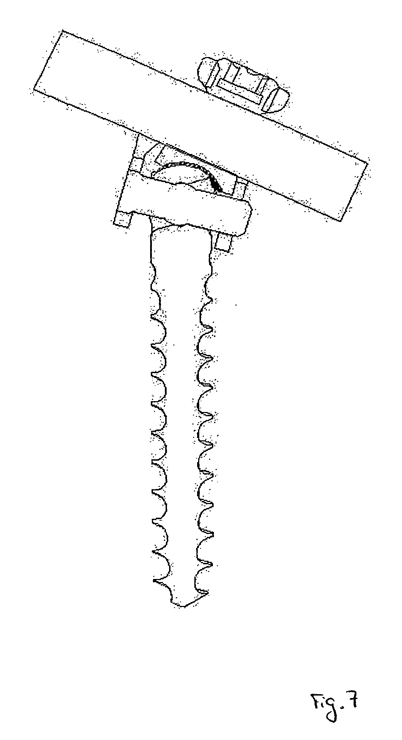

Le pivotement sagittal est représenté sur les figures 6 et 7, illustrant des vues en coupe de face du dispositif de connexion (1) selon l'invention avec une tige de liaison (2). Le pivotement sagittal peut s'effectuer, comme précisé précédemment, selon deux mouvements indépendants ou combinés.

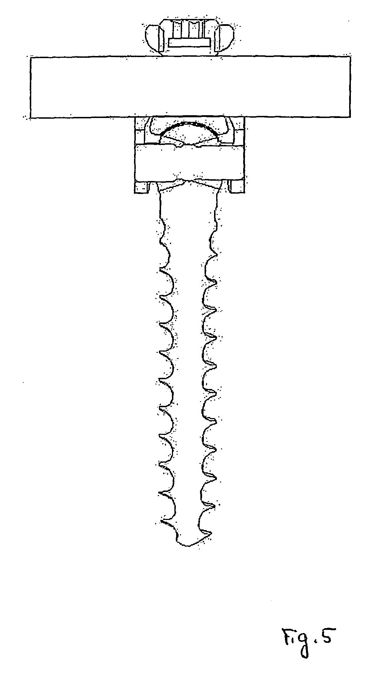

Afin de bien comprendre les deux mouvements sagittaux qui peuvent être effectués, il a été représenté sur la figure 5 une vue en coupe de face dudit dispositif de connexion (1) en position plane. Dans cette position, il apparaît que l'axe (6) traversant la tête (7) de la vis (3) présente une direction axiale parallèle à celle de la tige de liaison (2).

De cette position de repos, un premier mouvement de pivotement de ladite tige de liaison (2) peut être obtenu par le glissement de l'élément intermédiaire traversant (10) sur la tête (7) de ladite vis (3) sur lequel ledit élément intermédiaire traversant (10) repose (figure 6). Ce premier mouvement peut être complété, comme l'illustre la figure 7, par un second mouvement de pivotement lié au pivotement de la portion convexe (60) de l'axe (6) dans la cavité (8) de la tête (7) de la vis (3). De même que la direction axiale de la tige de liaison (2) avait été modifiée lors du premier mouvement de pivotement, il s'ensuit également un changement de la direction axiale dudit axe (6).

Il est bien entendu évident que, bien que non représenté, le mouvement de pivotement de l'axe (6) peut être réalisé indépendamment d'un mouvement de pivotement dudit élément intermédiaire traversant (10) et s'additionner à ce mouvement pour atteindre préférentiellement un débattement angulaire de 60 degrés.

Afin de contrôler les mouvements de pivotement, qu'il s'agisse du pivotement frontal ou des pivotements sagittaux, il sera avantageux de proposer des surfaces de liaison, non pas nécessairement lisses, mais présentant avantageusement des reliefs.

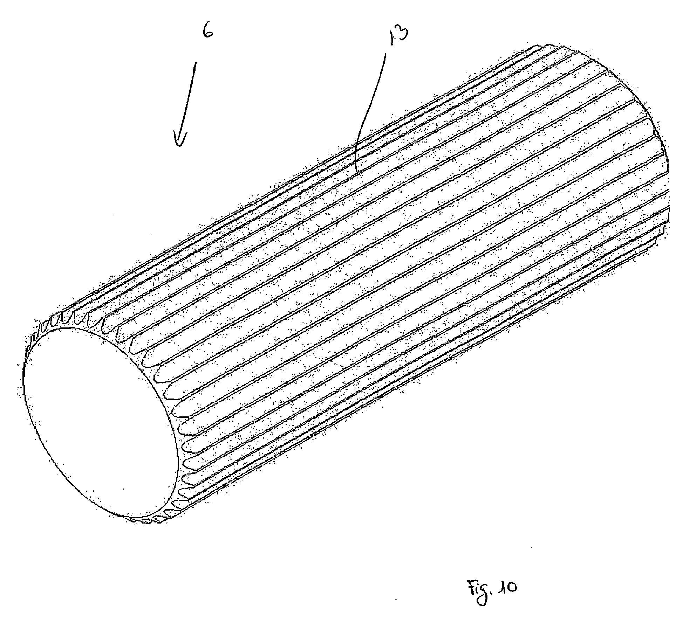

En particulier, il sera avantageux, en ce qui concerne le mouvement de pivotement frontal, d'utiliser un axe (6) pourvu de stries longitudinales (13). Celles-ci seront réparties, sinon sur toute sa surface, sur au moins la partie destinée à être disposée dans la cavité (7) de ladite vis (figure 10).

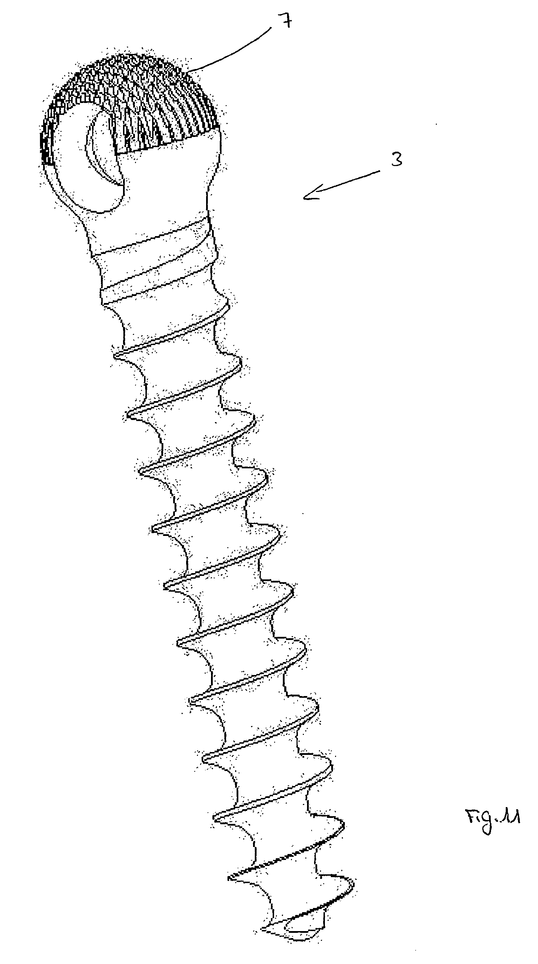

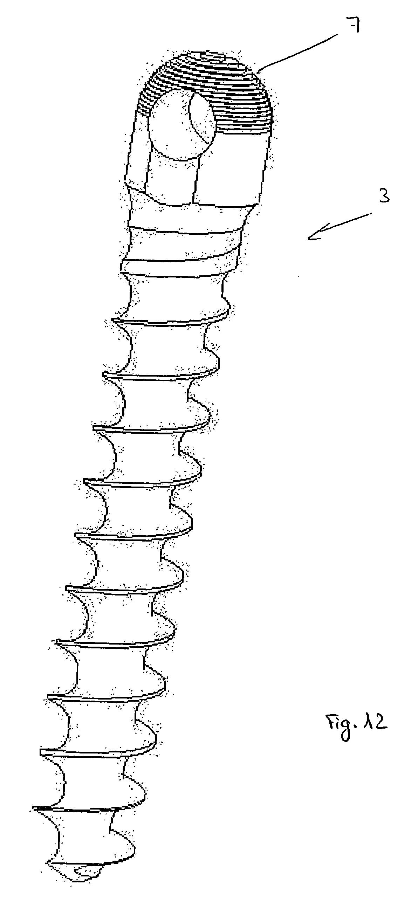

Il sera également avantageux que ladite vis (3) présente une tête (7) striée ou crantée comme illustré sur les figures 11 et 12.

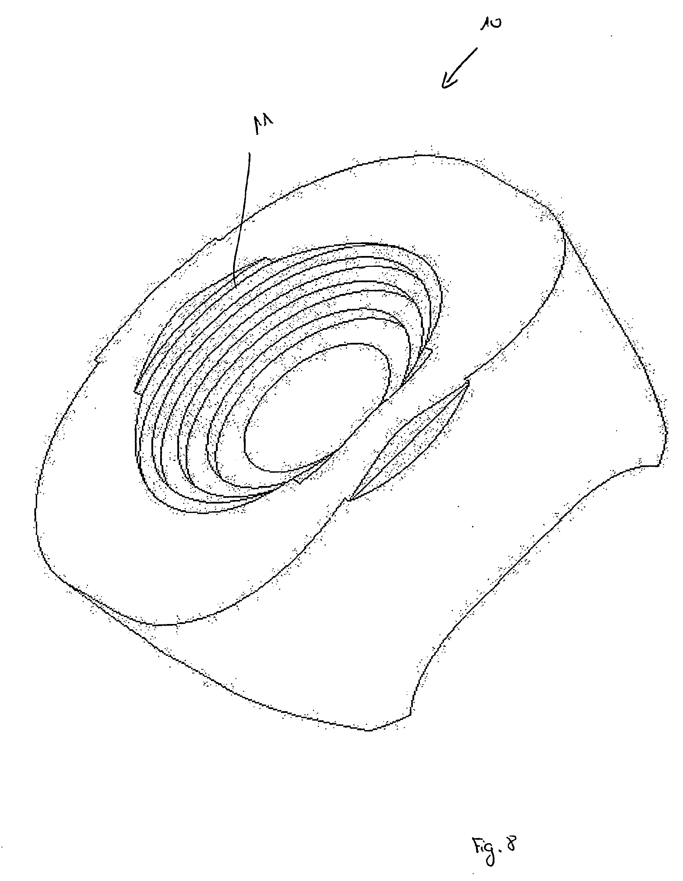

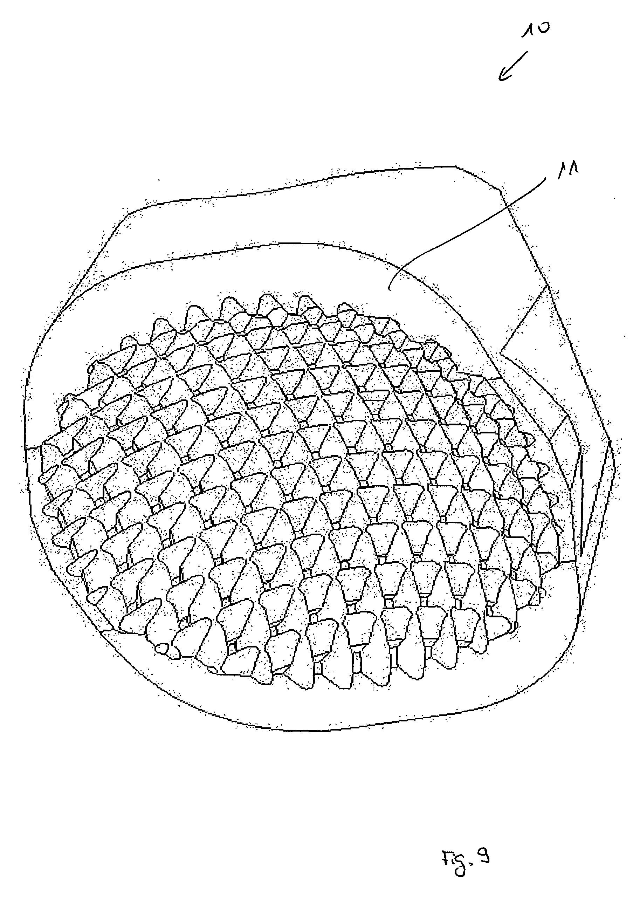

De même, l'élément intermédiaire traversant (10) pourra présenter sur la surface en contact avec la tête (7) de ladite vis (3) une zone crantée ou striée (figures 8 et 9).

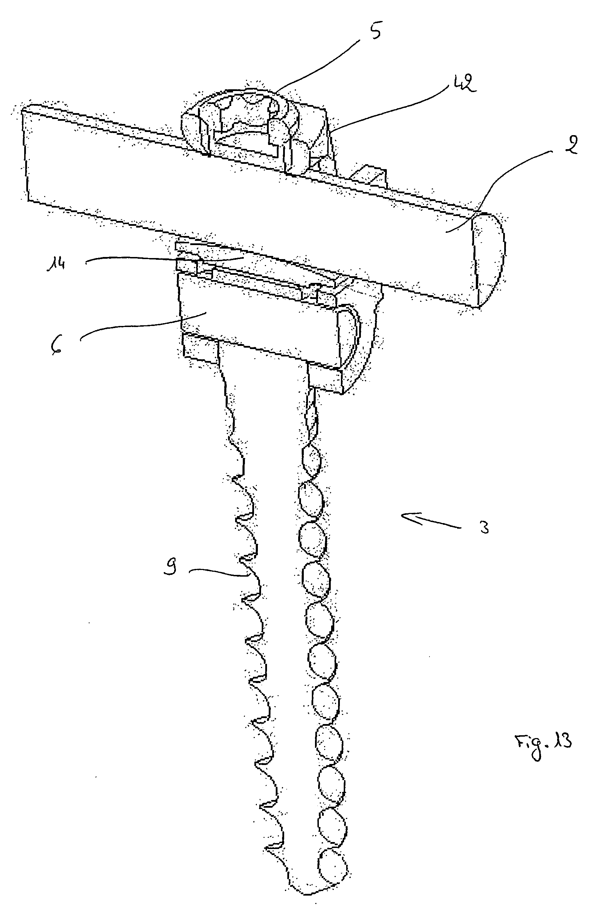

La figure 13 illustre une vue en coupe en perspective du dispositif de connexion (1) pourvue d'une tige de liaison (2) selon un autre mode de réalisation de l'invention.

Dans ce mode de réalisation, ledit dispositif de connexion (1) est constitué d'un moyen d'ancrage consistant en une vis (3), d'un connecteur (4) et d'un moyen de serrage (5).

Avantageusement, ladite pièce réceptrice (41) comprend une surface d'appui (14) bombée sur laquelle la tige de liaison (2) est disposée. Le mouvement de pivotement obtenu au moyen de l'élément intermédiaire traversant (10) dans le mode de réalisation précédent sera alors obtenu par le basculement de la tige de liaison (2) sur ladite surface d'appui (14). Dans ce mode de réalisation, la vis (3) reste donc libre en rotation frontale après serrage, et le cheminement des forces entre les éléments constitutifs du dispositif de connexion (1) est opéré comme suit : le moyen de serrage (5) exerce une pression sur la tige de liaison (2), laquelle exerce une pression sur la surface d'appui (14) de la surface réceptrice (41), laquelle exerce une traction sur la pièce de fermeture (42), elle-même exerçant une traction sur le bouchon de serrage (5).

L'invention est décrite dans ce qui précède à titre d'exemple. Il est entendu que l'homme du métier est à même de réaliser différentes variantes de l'invention sans pour autant sortir du cadre du brevet.

## Revendications

1. Dispositif de connexion (1) pour l'ostéosynthèse rachidienne comprenant un moyen d'ancrage (3) osseux, un connecteur (4) destiné à recevoir une tige de liaison (2) et assemblé avec une extrémité du moyen d'ancrage (3), ainsi qu'un moyen de serrage (5) pour immobiliser la tige de liaison (2), **caractérisé en ce que** l'assemblage du connecteur (4) et du moyen d'ancrage (3) est assuré par un axe (6) traversant le moyen d'ancrage (3), ledit axe (6) constituant une liaison pivot, et **en ce que** le dispositif (1) comporte un moyen de stabilisation, dans le plan contenant la tige et le moyen d'ancrage, du connecteur par rapport au moyen d'ancrage lorsqu'il est implanté sur le patient.

2. Dispositif de connexion (1) selon la revendication 1, **caractérisé en ce que** ledit moyen de stabilisation est constitué par le fait que ledit axe (6) présente une direction axiale parallèle à la direction axiale de ladite tige de liaison (2), autorisant un degré de rotation axial seulement, à l'exclusion d'un basculement dans le plan perpendiculaire à la tige.

3. Dispositif de connexion (1) selon la revendication 1, **caractérisé en ce que** ledit moyen de stabilisation est constitué par le fait que ledit axe (6) présente une direction axiale perpendiculaire à la direction axiale de ladite tige de liaison (2) et **en ce que** le dispositif comprend des moyens de blocage pour bloquer le mouvement de pivotement du connecteur (4) sur ledit moyen d'ancrage (3).

4. Dispositif de connexion (1) selon la revendication 1, **caractérisé en ce que** le connecteur (4) coopère avec ledit axe (6) avec un degré de rotation par rapport à la direction axiale, et un débattement angulaire par rapport à au moins une direction perpendiculaire.

5. Dispositif de connexion (1) pour l'ostéosynthèse rachidienne selon la revendication 4, **caractérisé en ce que** le connecteur (4) coopère avec ledit axe (6) avec une amplitude de rotation par rapport à la direction axiale comprise entre 90 et 180°.

6. Dispositif de connexion (1) pour l'ostéosynthèse rachidienne selon la revendication 4, **caractérisé en ce que** l'amplitude du débattement angulaire par rapport à au moins une direction perpendiculaire est comprise entre 20 et 60°.

7. Dispositif de connexion (1) selon la revendication 1, **caractérisé en ce que** la partie de l'axe (6) destinée à être disposée dans ledit moyen d'ancrage (3) présente une convexité répartie uniformément sur la circonférence dudit axe (6) pour permettre un débattement angulaire dudit axe (6) sur ledit moyen d'ancrage (3) par rapport à une direction perpendiculaire audit axe (6).

8. Dispositif de connexion (1) selon la revendication 2, **caractérisé en ce que** la partie de l'axe (6) destinée à être disposée dans ledit moyen d'ancrage (3) présente une convexité répartie uniformément sur la circonférence dudit axe (6) pour permettre un débattement angulaire sagittal dudit axe (6) sur ledit moyen d'ancrage (3).

9. Dispositif de connexion (1) selon la revendication 3, **caractérisé en ce que** la partie de l'axe (6) destinée à être disposée dans ledit moyen d'ancrage (3) présente une convexité répartie uniformément sur la circonférence dudit axe (6) pour permettre un débattement angulaire frontal dudit axe (6) sur ledit moyen d'ancrage (3).

10. Dispositif de connexion (1) selon la revendication 2 ou 8, **caractérisé en ce que** ledit connecteur (4) comporte une surface d'appui (14) sur laquelle la tige de liaison (2) s'appuie, ladite surface d'appui (2) étant configurée de manière à permettre un mouvement angulaire sagittal supplémentaire de ladite tige de liaison (2) par rapport audit moyen d'ancrage (3).

11. Dispositif de connexion (1) selon la revendication précédente, **caractérisé en ce que** ladite surface d'appui (14) est de forme bombée.

12. Dispositif de connexion (1) selon la revendication 10, **caractérisé en ce que** ladite surface d'appui (14) consiste en un élément formant berceau disposé dans le connecteur (4), ledit élément formant berceau présentant un logement inférieur (11) coopérant avec le connecteur (4) de manière à permettre le mouvement angulaire sagittal supplémentaire, et un logement supérieur (12) destiné à recevoir la tige de liaison (2).

13. Dispositif de connexion (1) selon la revendication 2 ou 8, **caractérisé en ce que** le connecteur (4) est traversé par un élément intermédiaire (10) recevant ladite tige de liaison (2), ledit élément intermédiaire (10) traversant présentant un logement inférieur (11) coopérant avec l'extrémité dudit moyen d'ancrage (3) de manière à permettre le verrouillage de tous les degrés de liberté de rotation dudit dispositif (1).

14. Dispositif de connexion (1) selon la revendication précédente, **caractérisé en ce que** ledit élément intermédiaire (10) traversant présente une face supérieure de forme bombée permettant le mouvement angulaire sagittal de ladite tige de liaison (2) par rapport audit connecteur (4).

15. Dispositif de connexion (1) selon la revendication 11, **caractérisé en ce que** ledit élément traversant consiste en un élément traversant formant berceau (10) permettant le mouvement angulaire sagittal de ladite tige de liaison (2) par rapport audit connecteur (4).

16. Dispositif de connexion (1) selon l'une quelconque des revendications 13 à 15, **caractérisé en ce que** le logement inférieur dudit élément intermédiaire traversant (10) présente au moins une zone crantée ou striée.

17. Dispositif de connexion (1) selon l'une quelconque des revendications 13 à 16, **caractérisé en ce que** l'extrémité du moyen d'ancrage (3) est sur tout ou partie crantée ou striée.

18. Dispositif de connexion (1) selon la revendication 3 ou 9, **caractérisé en ce que** les moyens de blocage consistent en une pièce traversant le connecteur (4), ladite pièce traversante présentant une face inférieure constituée d'un logement coopérant avec l'extrémité dudit moyen d'ancrage (3) et une face supérieure recevant ladite tige de liaison (2), de manière à permettre le verrouillage de tous les degrés de liberté de rotation dudit dispositif (1).

19. Dispositif de connexion (1) selon la revendication précédente, **caractérisé en ce que** la face supérieure est de forme bombée.

20. Dispositif de connexion (1) selon la revendication 18, **caractérisé en ce que** la pièce traversante consiste en un élément formant berceau lequel présente sur sa face supérieure un logement recevant ladite tige de liaison (2).

21. Dispositif de connexion (1) selon l'une quelconque des revendications 18 à 20, **caractérisé en ce que** le logement de la face inférieure de ladite pièce traversante est au moins en partie crantée ou striée.

22. Dispositif de connexion (1) selon l'une quelconque des revendications 18 à 21, **caractérisé en ce que** l'extrémité dudit moyens d'ancrage (3) en contact avec la pièce traversante est au moins en partie crantée ou striée.

23. Dispositif de connexion (1) selon l'une quelconque des revendications précédentes, **caractérisé en ce que** ledit axe (6) présente sur tout ou partie de sa surface des stries longitudinales (13).

24. Dispositif de connexion (1) selon l'une quelconque des revendications précédentes, **caractérisé en ce que** ledit axe (6) traverse l'extrémité du moyen d'ancrage (3) et coopère avec deux trous (414) prévus dans des branches (410, 411) du connecteur (4) lesquels présentent des sections complémentaires à la section de l'axe (6), lesdites branches (410, 411) étant disposées de part et d'autre dudit axe (6).

25. Dispositif de connexion (1) selon l'une quelconque des revendications précédentes, **caractérisé en ce que** le moyen d'ancrage (3) est une vis (3) laquelle présente une partie filetée (9) et une tête sphérique (7) pourvue d'une cavité (8) pour recevoir en partie ledit axe (6) cylindrique.

26. Dispositif de connexion (1) selon l'une quelconque des revendications précédentes, **caractérisé en ce que** le connecteur (4) est constitué d'une pièce réceptrice (41) en forme sensiblement de U destinée à recevoir une extrémité dudit moyen d'ancrage (3) pourvu de l'axe (6) et la tige de liaison (2), et d'une pièce de fermeture (42) en forme de U inversé, ladite pièce de fermeture (42) venant s'emboîter sur la pièce réceptrice (41) pour maintenir la tige de liaison (2) sur la partie réceptrice (41) dudit connecteur (4) par l'intermédiaire dudit moyen de serrage (5).

27. Système pour ostéosynthèse sur la colonne vertébrale, en particulier pour la stabilisation des vertèbres comprenant au moins une tige de liaison (2) et au moins deux dispositifs de connexion (1) selon l'une quelconque des revendications précédentes, lesdits dispositifs de connexion (1) étant susceptibles d'être chacun ancrés dans une vertèbre.

## Claims

1. Connecting device (1) for spinal osteosynthesis, comprising osseous anchoring means (3), a connector (4) that accommodates a joining shaft (2) and is fixed to one end of the anchoring means (3), and tightening means (5) for immobilising the joining shaft (2), **characterised in that** the connector (4) is fixed to the anchoring means (3) with the aid of a pin (6) that penetrates the anchoring means (3), said pin (6) embodying a pivot joint, and **in that** the device (1) comprises means for stabilising the connector relative to the anchoring means when the device is implanted in the patient, said stabilizing means being located on the plane encompassing the shaft and the anchoring means.

2. Connecting device (1) according to claim 1, **characterised in that** said stabilising means are based on the fact that the axial direction of said pin (6) is parallel to the axial direction of said joining shaft (2), allowing a degree of axial rotation, with the exception of swinging in the perpendicular plane of the shaft.

3. Connecting device (1) according to claim 1, **characterised in that** said stabilising means are based on the fact that the axial direction of said pin (6) is perpendicular to the axial direction of said joining shaft (2) and **in that** the device comprises blocking means for blocking the pivoting movement of the connector (4) on said anchoring means (3).

4. Connecting device (1) according to claim 1, **characterised in that** the connector (4) cooperates with said pin (6) with a degree of rotation relative to the axial direction and an angular displacement relative to at least one perpendicular direction.

5. Connecting device (1) for spinal osteosynthesis according to claim 4, **characterised in that** the connector (4) cooperates with said pin (6) with an amplitude of rotation relative to the axial direction comprised between 90 and 180°.

6. Connecting device (1) for spinal osteosynthesis according to claim 4, **characterised in that** the amplitude of angular displacement relative to at least one perpendicular direction is comprised between 20 and 60°.

7. Connecting device (1) according to claim 1, **characterised in that** the part of the pin (6) designed to penetrate said anchoring means (3) has a convexity that is evenly distributed around the circumference of said pin (6) so as to allow an angular displacement of said pin (6) on said anchoring means (3) relative to a direction that is perpendicular to said pin (6).

8. Connecting device (1) according to claim 2, **characterised in that** the part of the pin (6) designed to penetrate said anchoring means (3) has a convexity that is evenly distributed around the circumference of said pin (6) so as to allow a sagittal angular displacement of said pin (6) on said anchoring means (3).

9. Connecting device (1) according to claim 3, **characterised in that** the part of the pin (6) designed to penetrate said anchoring means (3) has a convexity that is evenly distributed around the circumference of said pin (6) so as to allow a frontal angular displacement of said pin (6) on said anchoring means (3).

10. Connecting device according to claim 2 or 8, **characterised in that** said connector (4) comprises a bearing surface (14) against which the joining shaft (2) can rest, said bearing surface (14) being configured so as to allow an additional sagittal angular movement of said joining shaft (2) relative to said anchoring means (3).

11. Connecting device (1) according to the preceding claim, **characterised in that** said bearing surface has a convex shape.

12. Connecting device (1) according to claim 10, **characterised in that** said bearing surface (14) consists of a cradle-shaped element placed in the connector (4), said cradle-shaped element having a lower housing (11), which cooperates with the connector (4) so as to allow the additional sagittal angular movement, and an upper housing (12) designed to accommodate the joining shaft (2).

13. Connecting device (1) according to claim 2 or 8, **characterised in that** the connector (4) is penetrated by an intermediate element (10) accommodating said joining shaft (2), said penetrating intermediate element (10) having a lower housing (11) cooperating with the end of said anchoring means (3) so as to allow said device (1) to lock in every degree of freedom of rotation.

14. Connecting device (1) according to the preceding claim, **characterised in that** said penetrating intermediate element (10) has a convex upper face that allows the sagittal angular movement of said joining shaft (2) relative to said connector (4).

15. Connecting device (1) according to claim 11, **characterised in that** said penetrating intermediate element consists of a cradle-shaped penetrating element (10) allowing the sagittal angular movement of said joining shaft (2) relative to said connector (4).

16. Connecting device (1) according to any one of the claims from 13 to 15, **characterised in that** the lower housing of said penetrating intermediate element (10) has at least one notched or grooved area.

17. Connecting device (1) according to any one of the claims from 13 to 16, **characterised in that** the end of the anchoring means (3) is completely or partially notched or grooved.

18. Connecting device (1) according to claim 3 or 9, **characterised in that** the blocking means consist of a part that penetrates the connector (4), said penetrating part having a lower face consisting of a housing that cooperates with the end of said anchoring means (3) and an upper face accommodating said joining shaft (2), so as to allow said device (1) to lock in every degree of freedom of rotation.

19. Connecting device (1) according to the preceding claim, **characterised in that** the upper face is convex.

20. Connecting device (1) according to claim 18, **characterised in that** the penetrating part consists of a cradle-shaped element, with a housing on its upper face that can accommodate said joining shaft (2).

21. Connecting device (1) according to any one of the claims from 18 to 20, **characterised in that** the housing of the lower face of said penetrating part is at least partially notched or grooved.

22. Connecting device (1) according to any one of the claims from 18 to 21, **characterised in that** the end of said anchoring means (3) in contact with the penetrating part is at least partially notched or grooved.

23. Connecting device (1) according to any one of the preceding claims, **characterised in that** said pin (6) has longitudinal grooves (13) on all or part of its surface.

24. Connecting device (1) according to any one of the preceding claims, **characterised in that** said pin (6) penetrates the end of the anchoring means (3) and cooperates with two holes (414) made in the branches (410, 411) of the connector (4), with sections that complement the section of the pin (6), said branches (410, 411) being arranged on either side of said pin (6).

25. Connecting device (1) according to any one of the preceding claims, **characterised in that** the anchoring means (3) consist of a screw (3) with a threaded part (9) and a spherical head (7) provided with a cavity (8) that can partially accommodate said cylindrical pin (6).

26. Connecting device (1) according to any one of the preceding claims, **characterised in that** the connector (4) consists of an accommodating part (41), which is substantially U-shaped and accommodates the end of said anchoring means (3) that includes the pin (6) and the joining shaft (2), and a locking part (42) in an inverted U shape, said locking part (42) fitting onto the accommodating part (41) so as to hold the joining shaft (2) against the accommodating part (41) of said connector (4) by means of said tightening means (5).

27. System for spinal osteosynthesis, in particular for stabilising vertebrae, comprising at least one joining shaft (2) and at least two connecting devices (1) according to any one of the preceding claims, said connecting devices (1) each being able to anchor to a vertebra.

## Patentansprüche

1. Verbindungsvorrichtung (1) für eine Wirbelsäulenosteosynthese, die ein Knochenverankerungsmittel (3), einen Verbinder (4) zur Aufnahme einer Verbindungsstange (2), welcher mit einem Ende des Verankerungsmittel (3) verbunden ist, sowie ein Spannmittel (5) zum Blockieren der Verbindungsstange (2) umfasst, **dadurch gekennzeichnet, dass** die Verbindung des Verbinders (4) und des Verankerungsmittels (3) durch eine durch das Verankerungsmittel laufende Welle (6) gewährleistet wird, wobei besagte Welle eine Drehverbindung bildet, und **dadurch** dass die Vorrichtung (1) auf der Ebene mit der Stange und dem Verankerungsmittel ein Mittel zur Stabilisierung des Verbinders in Bezug auf das Verankerungsmittel umfasst, wenn es dem Patienten implantiert wird.

2. Verbindungsvorrichtung (1) gemäß Patentanspruch 1, **dadurch gekennzeichnet, dass** besagtes Stabilisierungsmittel **dadurch** entsteht, dass besagte Welle (6) eine axiale Richtung aufweist, die parallel zur axialen Richtung besagter Verbindungsstange (2) verläuft, und nur einen axialen Rotationsgrad ermöglicht, mit Ausnahme einer Kippbewegung in der senkrechten Ebene zur Stange.

3. Verbindungsvorrichtung (1) gemäß Patentanspruch 1, **dadurch gekennzeichnet, dass** besagtes Stabilisierungsmittel **dadurch** entsteht, dass besagte Welle (6) in axialer Richtung senkrecht zur axialen Richtung besagter Verbindungsstange (2) verläuft, und **dadurch**, dass die Vorrichtung Blockierungsmittel zum Blockieren der Drehbewegung des Verbinders (4) auf besagtem Verankerungsmittel (3) umfasst.

4. Verbindungsvorrichtung (1) gemäß Patentanspruch 1, **dadurch gekennzeichnet, dass** der Verbinder (4) mit besagter Welle (6) mit einem Rotationswinkel in Bezug auf die axiale Richtung und einem Ausschlag in Bezug auf mindestens eine senkrechte Richtung zusammenwirkt.

5. Verbindungsvorrichtung (1) für eine Wirbelsäulenosteosynthese gemäß Patentanspruch 4, **dadurch gekennzeichnet, dass** der Verbinder (4) mit besagter Welle (6) in einer Drehamplitude von 90 bis 180° in Bezug auf die axiale Richtung zusammenwirkt.

6. Verbindungsvorrichtung (1) für eine Wirbelsäulenosteosynthese gemäß Patentanspruch 4, **dadurch gekennzeichnet, dass** die Amplitude des Ausschlags in Bezug auf mindestens eine senkrechte Richtung zwischen 20° und 60° beträgt.

7. Verbindungsvorrichtung (1) gemäß Patentanspruch 1, **dadurch gekennzeichnet, dass** der Teil der Welle (6), der dazu bestimmt ist, in besagtem Verankerungsmittel (3) angeordnet zu werden, eine gleichmäßig auf dem Umfang besagter Welle (6) verteilte Konvexität aufweist, um einen Ausschlag besagter Welle (6) auf besagtem Verankerungsmittel (3) in Bezug auf eine senkrechte Richtung zu dieser Welle (6) zu ermöglichen.

8. Verbindungsvorrichtung (1) gemäß Patentanspruch 2, **dadurch gekennzeichnet, dass** der Teil der Welle (6), der dazu bestimmt ist, in besagtem Verankerungsmittel (3) angeordnet zu werden, eine gleichmäßig auf den Umfang der Welle (6) verteilte Konvexität aufweist, um einen Ausschlag besagter Welle (6) in sagittaler Ebene auf besagtem Verankerungsmittel (3) zu ermöglichen.

9. Verbindungsvorrichtung (1) gemäß Patentanspruch 3, **dadurch gekennzeichnet, dass** der Teil der Welle (6), der dazu bestimmt ist, in besagtem Verankerungsmittel (3) angeordnet zu werden, eine gleichmäßig über den Umfang besagter Welle (6) verteile Konvexität aufweist, um einen frontalen Ausschlag besagter Welle (6) auf besagtem Verankerungsmittel (3) zu ermöglichen.

10. Verbindungsvorrichtung (1) gemäß Patentanspruch 2 oder 8, **dadurch gekennzeichnet, dass** besagter Verbinder (4) eine Auflagefläche (14) aufweist, auf der die Verbindungsstange (2) aufliegt, wobei besagte Auflagefläche (2) so gestaltet ist, dass eine zusätzliche Drehbewegung besagter Verbindungsstange (2) auf der sagittalen Ebene in Bezug auf das Verbindungsmittel (3) möglich ist.

11. Verbindungsvorrichtung (1) gemäß dem vorgenannten Patentanspruch, **dadurch gekennzeichnet, dass** besagte Auflagefläche (14) eine gewölbte Form hat.

12. Verbindungsvorrichtung (1) gemäß Patentanspruch 10, **dadurch gekennzeichnet, dass** besagte Auflagefläche (14) aus einem Element besteht, dass eine Lagerung bildet, die im Verbinder (4) angeordnet ist, wobei besagtes Element das eine Lagerung bildet, eine innere Aufnahme (11) aufweist, die mit dem Verbinder (4) so zusammenwirkt, dass die zusätzliche Drehbewegung in sagittaler Ebene möglich ist, und eine obere Aufnahme (12), die zur Aufnahme der Verbindungsstange (2) dient.

13. Verbindungsvorrichtung (1) gemäß Patentanspruch 2 oder 8, **dadurch gekennzeichnet, dass** durch Verbinder (4) ein Zwischenstück (10) verläuft, das besagte Verbindungsstange (2) aufnimmt, wobei besagtes quer verlaufendes Zwischenstück (10) eine untere Aufnahme (11) aufweist, die mit dem Ende besagten Verankerungsmittels (3) so zusammenwirkt, dass die Verriegelung aller Freiheitsgrade der Drehung besagter Vorrichtung (1) möglich ist.

14. Verbindungsmittel (1) gemäß dem vorhergehenden Anspruch, **dadurch gekennzeichnet, dass** besagtes quer verlaufendes Zwischenstück (10) eine gewölbte Oberseite aufweist, die die Drehbewegung besagter Verbindungsstange (2) auf sagittaler Ebene in Bezug auf besagten Verbinder (4) ermöglicht.

15. Verbindungsvorrichtung (1) gemäß Patentanspruch 11, **dadurch gekennzeichnet, dass** besagtes quer verlaufendes Stück aus einem quer verlaufenden Stück besteht, dass eine Lagerung (10) bildet, die eine Drehbewegung besagter Verbindungsstange (2) in Bezug auf besagten Verbinder (4) in sagittaler Ebene ermöglicht.

16. Verbindungsvorrichtung (1) gemäß einem der Patentansprüche 13 bis 15, **dadurch gekennzeichnet, dass** die untere Aufnahme besagten quer verlaufenden Zwischenstücks (10) mindestens einen gezahnten oder gerippten Bereich aufweist.

17. Verbindungsvorrichtung (1) gemäß einem der Patentansprüche 13 bis 16, **dadurch gekennzeichnet, dass** das Ende des Verankerungsmittels (3) überall oder teilweise gezahnt oder gerippt ist.

18. Verbindungsvorrichtung (1) gemäß Patentanspruch 3 oder 9, **dadurch gekennzeichnet, dass** die Blockierungsmittel aus einem Stück bestehen, das durch den Verbinder (4) läuft, wobei besagtes durchquerendes Stück eine Unterseite mit einer Aufnahme aufweist, die mit dem Ende des besagten Verankerungsmittels (3) zusammenwirkt, und eine Oberseite, die besagte Verbindungsstange (2) aufnimmt, so dass die Verriegelung aller Freiheitsgrade der Rotation besagter Vorrichtung (1) verriegelt ist.

19. Verbindungsvorrichtung (1) gemäß dem vorhergehenden Patentanspruch, **dadurch gekennzeichnet, dass** die Oberseite eine gewölbte Form aufweist.

20. Verbindungsvorrichtung (1) gemäß Patentanspruch 18, **dadurch gekennzeichnet, dass** das quer verlaufende Stück aus einem Element besteht, das eine Lagerung bildet und auf seiner Oberseite eine Aufnahme für besagte Verbindungsstange (2) aufweist.

21. Verbindungsvorrichtung (1) gemäß einem der Patentansprüche 18 bis 20, **dadurch gekennzeichnet, dass** die Aufnahme auf der Unterseite besagten quer verlaufenden Stücks mindestens teilweise gezahnt oder gerippt ist.

22. Verbindungsvorrichtung (1) gemäß einem der Patentansprüche 18 bis 21, **dadurch gekennzeichnet, dass** das Ende des besagten Verankerungsmittels (3), das mit dem quer verlaufenden Stück in Kontakt ist, mindestens zum Teil gezahnt oder gerippt ist.

23. Verbindungsvorrichtung (1) gemäß einem der vorhergehenden Patentansprüche, **dadurch gekennzeichnet, dass** besagte Welle (6) auf der gesamten Fläche oder auf Teilen davon längliche Rippen (134) aufweist.

24. Verbindungsvorrichtung (1) gemäß einem der vorhergehenden Patentansprüche, **dadurch gekennzeichnet, dass** besagte Welle (6) durch das Ende des Verankerungsmittels (3) geführt wird und mit zwei Löchern (414) zusammenwirkt, die in den Armen (410, 411) des Verbinders (4) vorgesehen sind und Querschnitte in Ergänzung zum Querschnitt der Welle (6) aufweisen, wobei besagte Arme (410, 411) zu beiden Seiten besagter Welle (6) angeordnet sind.

25. Verbindungsvorrichtung (1) gemäß einem der vorhergehenden Patentansprüche, **dadurch gekennzeichnet, dass** das Verankerungsmittel (3) eine Schraube (3) mit einem Gewindeteil (9) und einem kugelförmigen Kopf (7) mit einer Aushöhlung (8) zur Aufnahme eines Teils besagter zylindrischer Welle (6) ist.

26. Verbindungsvorrichtung (1) gemäß einem der vorhergehenden Patentansprüche, **dadurch gekennzeichnet, dass** der Verbinder (4) aus einem aufnehmenden Teil (41) mit deutlicher U-Form besteht, das dazu bestimmt ist, ein Ende des besagten Verankerungsmittels (3) mit der Welle (6) und der Verbindungsstange (2) aufzunehmen, und einem Verschlussteil (42) in umgekehrter U-Form, wobei das Verschlussteil (42) in das aufnehmende Teil (41) geschoben wird, um die Verbindungsstange (2) mittels der besagten Spannvorrichtung (5) auf dem aufnehmenden Teil (41) des besagten Verbinders (4) zu halten.

27. Osteosynthesesystem für die Wirbelsäule, teilweise zur Stabilisierung der Wirbel, das mindestens eine Verbindungsstange (2) und mindestens zwei Verbindungsvorrichtungen (1) gemäß einem der vorhergehenden Ansprüche aufweist, wobei besagte Verbindungsvorrichtungen (1) jeweils in einem Wirbel verankert werden können.
